# EUROPÄISCHE PATENTSCHRIFT

(11) **EP 3 000 445 B1**
(45) Veröffentlichungstag und Bekanntmachung des Hinweises auf die Patenterteilung: **02.08.2023**
(21) Anmeldenummer: 15189454.0
(22) Anmeldetag: 07.09.2011
(51) Int. Cl.: A61F 2/95, A61F 2/90

(54) **IMPLANTAT ZUR BEEINFLUSSUNG DES BLUTFLUSSES BEI ARTERIOVENÖSEN FEHLBILDUNGEN**
IMPLANT FOR INFLUENCING THE BLOOD FLOW RELATED TO ARTERIOVENOUS DEFECTS
IMPLANT DESTINE A INFLUENCER LA CIRCULATION SANGUINE PAR MALFORMATIONS ARTERIO-VEINEUSES

(30) Priorität: 08.09.2010 DE 102010044746
(43) Veröffentlichungstag der Anmeldung: 30.03.2016
(62) Teilanmeldung aus: 11764675.2
(73) Patentinhaber: Phenox GmbH, 44801 Bochum (DE)
(72) Erfinder: Schneider, Manuel, 44801 Bochum (DE); Rolla, Stefan, 44869 Bochum (DE); Aporta, Carsten, 44805 Bochum (DE); Hannes, Ralf, 44137 Dortmund (DE); Monstadt, Hermann, 44797 Bochum (DE)
(74) Vertreter: Schöneborn, Holger

(56) Entgegenhaltungen:
- US-A- 6 083 257
- US-A1- 2002 188 341
- US-A1- 2005 090 893
- US-A1- 2008 221 666
- US-A1- 2009 163 780
- US-A1- 2010 152 834

## Beschreibung

Die Erfindung betrifft ein Implantat für Blutgefäße, insbesondere zur Beeinflussung des Blutflusses im Bereich arteriovenöser Fehlbildungen, mit einer Wandung aus einzelnen Filamenten, die zu einem im Wesentlichen röhrenförmigen Geflecht zusammengefasst sind, das sich in axialer Richtung von proximal nach distal erstreckt, wobei die einzelnen Filamente sich gegenseitig kreuzen und miteinander Kreuzungspunkte bilden, das Implantat in der Weise verformbar ist, dass es in einem Einführungskatheter eine Form mit vermindertem Durchmesser annimmt und am Ort der Implantation unter Anpassung an den Blutgefäßdurchmesser expandiert, und am proximalen und/oder distalen Ende des Geflechts die Filamentenden jeweils mindestens paarweise zusammengeführt und miteinander dauerhaft verbunden sind, wobei die miteinander verbundenen Filamentenden atraumatisch geformt sind. Das Implantat ist insbesondere dazu bestimmt, den Blutfluss im Bereich von arteriovenösen Fehlbildungen, etwa Fisteln und Aneurysmen, zu beeinflussen. Es kann ferner auch zur Behandlung des ischämischen Schlaganfalls eingesetzt werden, etwa zur Wiederherstellung, Erhöhung oder Aufrechterhaltung des Blutflusses. Das Implantat kann rückholbar ausgelegt sein.

Arteriovenöse Fehlbildungen können in einem Patienten zu erheblichen Beeinträchtigungen und Gefährdungen bis hin zum Tode führen. Dies gilt insbesondere auch für arteriovenöse Fisteln und Aneurysmen, insbesondere dann, wenn sie im zerebralen Bereich auftreten. In der Regel versucht man derartige Fehlbildungen durch Implantate zu verschließen. Derartige Implantate werden in der Regel auf endovaskulärem Weg mit Hilfe von Kathetern gesetzt.

Insbesondere bei Aneurysmen hat sich die Implantierung von Platinspiralen bewährt, die das Aneurysma mehr oder weniger vollständig ausfüllen, den Bluteinstrom weitgehend blockieren und dazu führen, dass sich ein lokaler Thrombus ausbildet, der das Aneurysma ausfüllt und letztlich verschließt. Diese Behandlungsmethode ist allerdings nur bei Aneurysmen geeignet, die über einen relativ engen Zugang zum Gefäßsystem verfügen, sogenannte Beerenaneurysmen. Bei Aussackungen von Blutgefäßen, die über einen weiten Zugang zum Gefäß verfügen, drohen die implantierten Spiralen wieder ausgeschwemmt zu werden und Schäden in anderen Bereichen des Gefäßsystems herbeizuführen.

In solchen Fällen wurde bereits vorgeschlagen, eine Art Stent zu setzen, der die Öffnung des Aneurysmas "vergittert" und dadurch die Ausschwemmung der Okklusionsspiralen verhindert. Derartige Stents, die über eine relativ weitmaschige Wandung verfügen, haben aber eine Reihe von Nachteilen.

Zum einen ist dies die weitmaschige Struktur, die den Blutzutritt in das Aneurysma unbeeinträchtigt zulässt. Ist das Aneurysma aber nicht hinreichend mit dem Okklusionsmittel ausgefüllt, bleibt der Druck auf die Gefäßwandung unvermindert bestehen. Eine Nachbehandlung ist aber unter diesen Umständen nur schwer möglich, da der Stent den Zugang zum Aneurysma beeinträchtigt und die Einbringung weiterer Okklusionsmittel behindert.

Ein weiterer Nachteil ist die fehlende Anpassbarkeit des Stents an seinen Einsatzort. Für eine optimale Funktion sollte sich der Stent dicht an die Gefäßwandung anlegen, ohne jedoch einen übermäßigen Druck auf die Wandung auszuüben. Im Gegensatz zu Stents, die eine Aufweitung des Gefäßes bei Stenosen bewirken sollen, sind diese Stents eher als eine Art Manschette zu verstehen, die das Gefäßlumen und die Endothelwand des Gefäßes möglichst wenig beeinflussen soll. In der Folge sind diese Stents, selbst wenn sie für den Einsatzzweck speziell ausgewählt wurden, nur eingeschränkt an die Anforderungen angepasst.

Aus Drahtgeflecht bestehende Stents sind insbesondere für den Einsatz im koronaren Bereich seit langem bekannt. Diese Stents werden in der Regel als Rundgeflecht gefertigt, wobei die einzelnen Drahtfilamente in gegenläufigen spiralförmigen bzw. helixförmigen Lagen die Stentwandung ausbilden. Es entsteht ein Maschengeflecht, das in radialer Richtung sowohl abstützt als auch für Blut durchlässig ist.

Ein Problem dieser als Rundgeflecht ausgebildeten Stents sind die an den freien Enden bestehenden losen Enden, die aufgrund ihres geringen Durchmessers, traumatisch wirken können.

Gemäß US-A-4 655 771 (Wallsten) wird ein solcher als Rundgeflecht ausgebildeter Stent in seinen Endbereichen durch U-förmige Verbindungsglieder zwischen den losen Enden atraumatisch gestaltet. Die U-förmigen Verbindungsglieder führen allerdings dazu, dass Spannungen entstehen, die zu einer Deformierung des Stents führen.

Gemäß US-A-5 061 275 (Wallsten et al.) werden die losen Enden derartiger Drahtstents durch Laserbehandlung rundgeschmolzen, um so einer Traumatisierung entgegenzuwirken. Der dort beschriebene Stent besteht ebenfalls aus einem Rundgeflecht, bei dem die einzelnen Drähte im Bereich der Knoten Einprägungen aufweisen, um eine spannungsfreie Fixierung innerhalb der Wandung zu ermöglichen.

Gemäß US-A-6 083 257 werden Filamentenden des Stents jeweils paarweise zusammengeführt und verbunden.

Derartige als Rundgeflecht aus Filamenten bestehende Stents werden häufig beim Einsatz zur Behandlung von Stenosen mit Hilfe eines Ballons am Einsatzort hydraulisch aufgeweitet und an der Gefäßwandung fixiert. Während der Einbringung dient der an einem Einführdraht befestigte Ballon als Transportvehikel, auf das der Stent aufgecrimpt ist. Ein solches Transportvehikel sollte aber für Implantate, die zur Beeinflussung bzw. Kanalisierung des Blutflusses im zerebralen Bereich dienen, nicht eingesetzt werden; vielmehr ist hier ein Implantat, das sich selbständig an den Gefäßdurchmesser anpasst und an die Gefäßwandung anlegt, von Vorteil.

Ein weiteres Problem der aus Drahtgeflecht bestehenden Stents oder Implantate ist die Fertigung. Vorteilhaft ist die Fertigung als geflochtener Endlosschlauch, der auf die gewünschte Dimension abgelängt wird. Hierbei entstehen im Bereich der beiden Enden des abgelängten Schlauches lose Drahtenden, die aufwändig entschärft werden müssen, beispielsweise durch die oben erwähnten Verbindungsglieder.

In der WO 2008/107172 A1 wird ein Implantat beschrieben, bei dem das Geflecht in einem Einführungskatheter eine gelängte Form mit vermindertem Durchmesser aufweist und am Implantationsort unter Anpassung an den Gefäßdurchmesser und Zunahme der Geflechtdichte expandiert, wobei die an den Implantatenden herausstehenden Filamentenden zumindest paarweise zusammengeführt und miteinander verbunden sind. Auf diese Weise wurde ein Implantat zur Verfügung gestellt, das in der Lage ist, sich an den jeweiligen Gefäßdurchmesser anzupassen, wobei die Filamentenden atraumatisch sind.

Aus der DE 10 2009 006 180 A1 ist ein Implantat bekannt, das sich ebenfalls atraumatische Eigenschaften zum Ziel gesetzt hat. Dabei werden die Drahtenden zu ersten Geflechtenden und diese wiederum zu zweiten Geflechtenden zusammengeführt.

Die US 2008/0221666 A1 zeigt ein Stent-Ablösesystem, bei dem ein Draht helikal um ein Ablöseelement gewunden ist, wobei der Draht einen elektrolytisch korrodierbaren Abschnitt aufweist.

Der Erfindung liegt die Aufgabe zugrunde, ein Implantat der eingangs genannten Art bzw. eine Kombination aus Implantat und Einführdraht zur Verfügung zu stellen, die eine sichere und kontrollierte Ablösung des Implantats gewährleistet.

Diese Aufgabe wird erfindungsgemäß gelöst durch eine Kombination nach Anspruch 1.

Am proximalen Ende des Implantats befinden sich Verdickungen, die vom Halteelement formschlüssig gehalten werden, wobei ein Abschnitt des Halteelements elektrolytisch korrodierbar ausgebildet ist, so dass nach elektrolytischer Auflösung des Abschnitts das proximale Ende des Implantats freigegeben wird. In diesem Fall wird die Ablösung des Implantats nicht lediglich durch Ausschieben aus dem Katheter bzw. Zurückziehen einer Abdeckung herbeigeführt, stattdessen ist zumindest auch die elektrolytische Korrosion eines Abschnitts des Halteelements notwendig. Der korrodierbare Abschnitt ist dabei so angeordnet, dass er den Austritt der in das Halteelement hineinragenden Verdickungen verhindert. Es kann sich z. B. um einen Stift handeln, der zwischen den Verdickungen angeordnet ist und diese auseinanderhält, so dass der Durchmesser des Implantats am proximalen Ende für einen Austritt aus dem Halteelement zu groß ist. Die Festlegung des Implantats am Halteelement über Formschluss, der über die elektrolytische Ablösbarkeit eines Abschnitts des Halteelements kontrolliert wird, ist im Hinblick auf die sichere Steuerbarkeit der Platzierung und ggf. auch die Repositionierung oder das Zurückziehen des Implantats besonders vorteilhaft.

Eine weitere Möglichkeit besteht darin, als korrodierbar ausgebildeten Abschnitt des Halteelements eine Scheibe mit einer Öffnung zu verwenden, wobei sich die am proximalen Ende des Implantats befindlichen Verdickungen durch die Öffnung erstrecken und wobei der Durchmesser der Öffnung so auf die Verdickungen abgestimmt ist, dass ein Durchtreten der Verdickungen durch die Öffnung bei intakter Scheibe ausgeschlossen ist. Erst wenn sich die Scheibe durch Anlegen einer Spannung zumindest teilweise auflöst, können die Verdickungen des Implantats aus dem Halteelement austreten.

Für den elektrolytisch korrodierbaren Abschnitt können verschiedene Materialien verwendet werden, die eine rasche Ablösbarkeit gewährleisten und darüber hinaus medizinisch verträglich sind. Beispiele sind Edelstahl, Magnesium, Magnesiumlegierungen oder Cobalt-Chrom-Legierungen.

Bei Implantaten hat sich als Problem herausgestellt, dass sich die Filamentenden am proximalen Ende bei der Freisetzung des Implantats manchmal nicht in ausreichendem Maße zu den Gefäßwänden hin aufweiten. Dieser Effekt wird in der Literatur auch teilweise als "Fischmauleffekt" bezeichnet und ist u. a. darauf zurückzuführen, dass am proximalen Ende des Implantats geringere Radialkräfte wirken als am distalen Ende. Bei der Freisetzung wird zunächst die über dem Implantat liegende Abdeckung entfernt, so dass sich das distale Ende aufweiten kann, während das proximale Ende weiterhin mit dem Ablösemechanismus in Verbindung steht. Erst nach Freisetzung vom Ablösemechanismus kann auch das proximale Ende expandieren. Die Kräfte, die das proximale Ende radial nach außen drücken, sind hier jedoch geringer als am distalen Ende und im mittleren Bereich, weil die Expansion ohne zusätzliche Unterstützung erfolgen muss.

Der Fischmauleffekt hat zur Folge, dass Filamentenden am proximalen Ende in das Gefäßlumen hineinragen und für eine Behinderung des Blutflusses sorgen. Das Implantat ist somit unter Umständen nicht in ausreichendem Maße atraumatisch. Weitere Interventionen, bei denen z. B. ein Katheter das Implantat passieren muss, werden erschwert oder behindert.

Zur Lösung dieses Problems können die sich kreuzenden Filamente an den distal der Filamentenden liegenden Kreuzungspunkten am proximalen Ende des Implantats jeweils direkt oder indirekt miteinander verbunden sein.

Es hat sich herausgestellt, dass die gegenseitige Fixierung der Filamente aneinander im Bereich der Kreuzungspunkte am proximalen Ende dazu führt, dass sich das Implantat auch an diesem Ende in ausreichendem Maße radial aufweitet, so dass die Filamentenden eng an der Gefäßwand anliegen. Die mangelnde radiale Aufweitung der Filamente bei den beschriebenen Implantaten aus dem Stand der Technik wird u. a. darauf zurückgeführt, dass an den Kreuzungspunkten zwischen den Filamenten eine Reibung entsteht, die die Aufweitung behindert. Diese Reibung wird durch die gegenseitige Fixierung der Filamente im Bereich der Kreuzungspunkte unterbunden. Die Kreuzungspunkte werden vielmehr in einer bestimmten axialen Position relativ zum proximalen Ende des Implantats gehalten, so dass keine Verlagerung einzelner Kreuzungspunkte verbunden mit einer gegenseitigen Behinderung der Filamente bei der Aufweitung mehr auftritt.

Unter den Kreuzungspunkten am proximalen Ende des Implantats werden die Kreuzungspunkte verstanden, an denen sich die Filamente zwar kreuzen, nicht aber die Filamentenden selbst, an denen die Filamente dauerhaft miteinander verbunden sind. Die proximal gelegenen Kreuzungspunkte liegen somit vom proximalen Filamentende aus gesehen geringfügig in Richtung distal. Erfindungsgemäß werden unter Kreuzungspunkten am proximalen Ende des Implantats die Kreuzungspunkte in den ersten drei orthogonal zur Längsrichtung des Implantats liegenden Kreuzungspunktebenen verstanden, d. h. die am weitesten proximal liegenden Kreuzungspunkte, die am zweitweitesten proximal liegenden Kreuzungspunkte und die am drittweitesten proximal liegenden Kreuzungspunkte. Bevorzugt sind zumindest die sich kreuzenden Filamente an den am weitesten proximal liegenden Kreuzungspunkten miteinander verbunden, es kann jedoch sinnvoll sein, auch die sich kreuzenden Filamente an den etwas weiter distal liegenden Kreuzungspunkten zu verbinden. Denkbar ist auch eine Verbindung von sich kreuzenden Filamenten an Kreuzungspunkten im distalen Bereich des Implantats.

Die Verbindung der Filamente untereinander an den proximal gelegenen Kreuzungspunkten kann auf unterschiedliche Weise erfolgen. Eine Möglichkeit besteht darin, die Filamente durch Schlaufen zu führen, die durch separate Drähte gebildet werden. Diese Drähte sind am Implantat festgelegt, vorzugsweise am proximalen Ende des Implantats. Die sich im Kreuzungspunkt überschneidenden Filamente werden durch die Schlaufen geführt, so dass der Kreuzungspunkt in seiner axialen Lage weitgehend fixiert ist und die Filamente nicht aneinander vorbeigleiten können. Reibung zwischen den Filamenten wird somit unterbunden. Andererseits haben die Filamente einander gegenüber noch eine, wenn auch stark eingeschränkte, Bewegungsfreiheit, so dass die Aufweitung des Implantats nicht behindert wird.

Die die Schlaufen bildenden Drähte können insbesondere von den Punkten ausgehen, an denen die Filamente am proximalen Ende zusammengeführt sind. Eine Verbindung ist bspw. mittels Laserschweißen, Hartlöten, Kleben o. ä. möglich. Die einzelnen Drähte erstrecken sich vom proximalen Festlegungspunkt in Richtung distal und werden um die proximalen Kreuzungspunkte der Filamente herumgelegt.

Alternativ können auch zumindest einige der Filamente selbst an den am weitesten proximal liegenden Kreuzungspunkten Ösen bilden, durch die jeweils ein oder mehrere andere Filamente verlaufen, die sich an diesem Kreuzungspunkt mit dem eine Öse aufweisenden Filament kreuzen. Dieses weitere Filament kann wiederum selbst an dieser Stelle eine Öse aufweisen, um die gegenseitige Fixierung zu gewährleisten. Bei dieser Ausführungsform ist ein separater Draht zur Fixierung der Filamente entbehrlich.

Weitere alternative Möglichkeiten der Verbindung der Filamente im Bereich der proximalen Kreuzungspunkte sind möglich. Insbesondere können die Filamente hier miteinander verknotet oder miteinander verklebt, verlötet oder verschweißt sein. Auch sonstige form- oder stoffschlüssige Arten der Verbindung sind denkbar. Die Verbindung sollte allerdings über ein gewisses Maß an Flexibilität verfügen, damit eine Änderung des Winkels, in dem sich die Filamente an den Kreuzungspunkten schneiden, weiterhin möglich ist.

Das erfindungsgemäße röhrenförmige Geflecht ist zumeist ein Rundgeflecht und hat einen vom proximalen oder distalen Ende aus betrachtet kreisförmigen Querschnitt. Grundsätzlich sind jedoch auch Abweichungen von der Kreisform möglich, beispielsweise ein ovaler Querschnitt.

Bei den Filamenten, die die Geflechtstruktur bilden, kann es sich um einzelne Drähte aus Metall handeln, möglich ist aber auch das Vorsehen von Litzen, d. h. mehreren Drähte geringen Durchmessers, die zusammen ein Filament bilden und vorzugsweise miteinander verdrillt sind.

Die erfindungsgemäßen Implantate sind in der Lage, den Blutfluss in einem Gefäß zu beeinflussen, dergestalt, dass arteriovenöse Fehlbildungen soweit wie möglich vom Blutfluss abgeschottet werden. Entsprechendes gilt für den Verschluss von Gefäßen, die beispielsweise vom Blutkreislauf abgekoppelt werden sollen, weil sie etwa Tumore versorgen. Das Implantat soll bei optimaler Auswahl von Implantatdurchmesser zu Gefäßdurchmesser dabei in der Lage sein, sich an den jeweiligen Gefäßdurchmesser anzupassen. Im Bereich von Erweiterungen und Aussackungen soll es maximal seinen Nenndurchmesser annehmen.

Das Implantat kann des Weiteren auf eine atraumatische Art und Weise platziert werden, d. h. ohne die Hilfe eines Ballons. Eine Platzierungsvorrichtung muss das Implantat bis zur endgültigen Freisetzung aus dem Katheter zuverlässig festhalten und sollte insbesondere auch das Zurückziehen des Implantates in den Katheter ermöglichen, solange noch keine vollständige Freisetzung erfolgt ist.

Als Material für das erfindungsgemäße Implantat kommen insbesondere Materialien mit hoher Rückstellkraft bzw. Federwirkung in Frage. Dies sind insbesondere Materialien mit superelastischen oder Formgedächtniseigenschaften, beispielsweise Nitinol. Dabei können für die einzelnen Filamente auch Drähte mit unterschiedlichem Durchmesser verwendet werden. Die Drähte mit größerem Durchmesser sorgen für eine ausreichende Radialkraft, die Drähte mit kleinerem Durchmesser bewirken eine ausreichend hohe Maschendichte. Die Vor- und Nachteile von Drähten mit unterschiedlichem Querschnitt können auf diese Weise kombiniert bzw. kompensiert werden. Der Querschnitt der Drähte ist in den meisten Fällen rund, möglich sind aber auch Drähte mit ovalem oder eckigem Querschnitt oder Kombinationen hieraus.

Im Rahmen der Beschreibung wird unter dem Begriff proximales Ende das Ende verstanden, dass dem behandelnden Arzt zugewandt ist, d. h. das proximale Ende weist in Richtung Körperäußeres. Umgekehrt ist das distale Ende dem Arzt abgewandt, geht also in Richtung Körperinneres. Entsprechend lässt sich proximal und distal als dem Einführdraht des Platzierungssystems zugewandt bzw. abgewandt verstehen.

Die erfindungsgemäßen Implantate werden anhand eines Geflechts zur Abschottung eines Aneurysmas beschrieben. Es versteht sich, dass derartige Geflechte für verschiedene Zwecke eingesetzt werden können, insbesondere für andere Formen von arteriovenösen Fehlbildungen.

Die erfindungsgemäßen Implantate müssen nicht unbedingt, wie es bei üblichen Stents der Fall ist, eine Stützfunktion ausüben. Sie dienen vielmehr in erster Linie zur Kanalisierung des Blutflusses im Bereich der Fehlbildungen. Sie sollen beispielsweise auch in einem Aneurysma platzierte Okklusionsmittel daran hindern, in die Gefäßbahn ausgeschwemmt zu werden. Darüber hinaus kann der Ein- und/oder Ausfluss von Blut in ein Aneurysma verhindert werden. Es handelt sich um eine Art Inliner, innere Manschette oder auch Flow Diverter. Grundsätzlich ist aber auch mit den erfindungsgemäßen Implantaten die Herbeiführung einer Stützfunktion im Sinne herkömmlicher Stents möglich.

Die erfindungsgemäßen Implantate werden als Geflecht aus einer Mehrzahl von Filamenten gefertigt, wobei das Geflecht im Prinzip einen Endlosschlauch bildet. Die jeweils benötigte Implantatlänge kann dann aus diesem Endlosschlauch abgelängt werden. Die einzelnen Filamente sind dazu spiral- oder helixförmig aufgewickelt, wobei die einzelnen Filamente als Flechtwerk, d. h. einander kreuzend untereinander und übereinander eingebracht werden. In der Regel sind die einzelnen Filamente dabei in zwei einander in einem konstanten Winkel kreuzenden Richtungen gewickelt, die sich beispielsweise in einem Winkel von 90° schneiden. Erfindungsgemäß bevorzugt sind - im spannungsfreien Normalzustand - Winkel von mehr als 90°, insbesondere von 90 bis 160°, wobei die zu den axialen Enden des Implantats hin offenen Winkel gemeint sind. Eine solche steile Wicklung der Einzelfilamente kann, wenn sie hinreichend dicht ist, zu einem Geflecht mit hoher Oberflächendichte führen, das bei axialer Streckung zu erheblich geringeren Durchmessern auseinandergezogen werden kann. Bei Wegfall der Streckkräfte und hinreichender Rückstellkraft des Filamentmaterials nähert sich das Geflecht dem Nenndurchmesser, d. h. dem ursprünglich spannungsfreien Zustand, wieder an und dehnt sich aus, was zu einer engen Anschmiegung an die Gefäßwand am Implantationsort und zu einer Verdichtung der Maschenstruktur an der Wandung führt. Dies gilt insbesondere auch im Bereich von Gefäßerweiterungen. Zusätzlich kann die Oberflächendichte des Geflechts auch durch die angewandte Flechttechnik variiert werden. Beispielsweise kann das Implantat im mittleren Bereich, in dem typischerweise das Aneurysma abgedeckt wird, dichter geflochten sein als in den Endbereichen, so dass eine weitgehende Abdeckung des Aneurysmahalses gewährleistet ist. Auf der anderen Seite wird durch eine geringere Oberflächendichte in den Endbereichen eine hinreichende Flexibilität gewährleistet.

In dem erfindungsgemäßen Geflecht werden die an den Implantatenden herausstehenden Filamentenden zumindest paarweise zusammengeführt und miteinander dauerhaft verbunden. Dies kann beispielsweise durch Verschweißen erfolgen, aber auch durch mechanisches Verklammern, Verdrillen, Verlöten oder Verkleben. Eine Verbindung der Filamentenden kann auch mittels einer aufgesetzten Hülse erfolgen. Diese Hülse kann mit den Filamentenden eine stoffschlüssige Verbindung eingehen, beispielsweise verschweißt oder auch vercrimpt sein. Eine Alternative besteht darin, dass die Hülse so dimensioniert ist, dass an den Filamentenden befindliche Verdickungen daran gehindert sind, durch die Hülse hindurchzurutschen. Die Hülse ist somit relativ zu den Filamenten in axialer Richtung verschiebbar, sie kann aber nicht vollständig abgezogen werden. Des Weiteren ist es vorteilhaft, wenn die Hülsen in axialer Richtung gegeneinander versetzt sind. Auf diese Weise wird erreicht, dass die Hülsen beim Komprimieren des Implantats nicht unmittelbar übereinander zu liegen kommen, so dass das Implantat insgesamt einen geringeren Durchmesser aufweist.

Möglich ist auch eine Zusammenführung der Filamente zu ersten Geflechtenden, die wiederum zu zweiten Geflechtenden verbunden sind, wie in der DE 10 2009 006 180 A1 beschrieben.

Eine weitere Möglichkeit besteht darin, an den Filamentenden Schlaufen vorzusehen, d. h. die Filamentenden werden am proximalen/distalen Ende zusammengeführt und um 180° zurückgebogen. Auf diese Weise werden die Filamentenden atraumatisch. Sie können über aufgestülpte Hülsen oder aufgesteckte Coils zusammengehalten werden. Die Verbindung erfolgt über vercrimpen, verkleben o. ä.

Dabei oder zusätzlich werden die verbundenen Filamentenden atraumatisch umgeformt. Insbesondere können die Filamentenden distal und proximal eine atraumatische Verdickung aufweisen, die z. B. ungefähr kugelförmig ist. Die Verdickung kann durch Laserschweißen, Hartlöten, Verkleben, Aufcrimpen o. ä. aus dem Filamentende ausgeformt oder am Filamentende angebracht werden.

Die Platzierung der erfindungsgemäßen Implantate wird in der Praxis unter Röntgenkontrolle erfolgen. Aus diesem Grund sollte das Implantat ein röntgendichtes Markermaterial aufweisen, sofern es nicht aus einem röntgendichten Material selbst gefertigt ist. Solche röntgendichten Materialien sind insbesondere Tantal, Gold, Wolfram und Platinmetalle, etwa Pt-Ir-Legierungen, wobei letztere bevorzugt sind. Diese Marker können beispielsweise als Markerelemente in bekannter Weise an die Filamentenden angeheftet werden, aber auch als Markerfilamente in die Geflechtstruktur des Implantates eingeflochten werden. Die Ummantelung einzelner Filamente mit einer Helix oder Draht aus einem röntgendichten Material wie Platin ist ebenfalls möglich. Die Helix bzw. der Draht kann mit den Filamenten verschweißt, verklebt o. ä. sein. Eine weitere Möglichkeit besteht in der Beschichtung oder Füllung der Filamente mit einem röntgendichten Material.

Eine weitere Alternative sind röntgendichte Markierungen in Form von Hülsen, die die zusammengeführten Filamente umschließen. Diese Hülsen können ebenfalls mit den Filamentenden verschweißt oder auch vercrimpt sein. Die röntgendichten Hülsen können mit den oben erwähnten Hülsen zum Zusammenhalten der Filamentenden identisch sein und somit eine Doppelfunktion erfüllen.

Die erfindungsgemäßen Implantate werden in der Regel nicht mit Hilfe eines Ballons hydraulisch geweitet und platziert. Gleichwohl ist es notwendig, die Implantate mit einem Einführdraht so zu verbinden, dass sie zuverlässig geführt werden können. Dies erfolgt erfindungsgemäß über Verbindungselemente in Form von Verdickungen, die mit einem Halteelement des zur Platzierung erforderlichen Einführdrahtes zusammenwirken. Als solche Verbindungselemente können die miteinander verbundenen Filamentenden des Geflechts vorgesehen sein.

Gefäßabzweigungen (Bifurkationen) können bei den erfindungsgemäßen Implantaten beispielsweise durch Bereiche einer geringeren Maschendichte berücksichtigt werden.

Das Geflecht kann im Prinzip auf jede bekannte Art und Weise geflochten werden. Es liegt ein- und/oder mehrflechtig vor. Eine enge Flechtigkeit führt insbesondere bei einem dichten Flechtwerk zu einer hohen Beanspruchung der einzelnen Filamente. Insoweit ist die mehrflechtige Ausführung geeignet, Spannung aus dem Flechtwerk zu nehmen, wobei aber eine zu hohe Flechtigkeit zu einem schlechten Verbund im Geflecht führt. Die Flechtigkeit gibt an, an wie vielen sich mit dem Filament kreuzenden Filamenten ein bestimmtes Filament auf derselben Seite vorbeigeführt wird, bevor es die Seite wechselt, um anschließend an einer entsprechenden Zahl von kreuzenden Filamenten auf der anderen Seite vorbeigeführt zu werden. Bei einer zweiflechtigen Ausführung z. B. wird ein Filament nacheinander oberhalb von zwei das Filament kreuzenden Filamenten, dann nacheinander unterhalb von zwei kreuzenden Filamenten geführt.

Die Filamente können insbesondere auch mehrfach gefacht sein. Die Fachung gibt die Anzahl der zusammengefassten, parallellaufenden Einzelfilamente an. Möglich ist eine Einfach- oder Mehrfachfachung, bei der jeweils ein oder mehrere Einzelfilamente parallel laufen. Da bei der Fertigung des Geflechts die Filamente von Spulen zugeführt werden, bedeutet dies, dass von der entsprechenden Spule ein bzw. mehrere Einzelfilamente gleichzeitig dem Dorn, auf dem das Geflecht gefertigt wird, zugeführt werden. Jedes Einzelfilament kann aus einem einzelnen Draht oder auch aus einer Litze von mehreren zusammengefassten und vorzugsweise miteinander verdrillten Einzeldrähten bestehen.

Die Einzeldrähte können den gleichen Durchmesser sowie auch unterschiedliche Durchmesser aufweisen. Auch können die Drähte aus unterschiedlichen Materialien (Nitinol, Cobalt-Chrom-Legierungen, Platin-Legierungen) bestehen. Drähte aus einem röntgendichten Material beispielsweise sorgen für die Röntgensichtbarkeit des Implantats.

Erfindungsgemäß sind die Filamentenden insbesondere paarweise miteinander verbunden, wobei bei Mehrfachfilamenten paarweise bedeutet, dass jeweils zwei Bündel aus mehreren Einzelfilamenten zusammengeführt werden. Diese Bündelung kann dabei kompakt erfolgen, dergestalt, dass alle Drähte zu einem im Wesentlichen runden Bündel zusammengefasst werden und die Stirnseiten aller Drähte gemeinsam aufgeschmolzen werden, so dass eine einheitliche Kuppel entsteht. Dadurch wird eine stoffschlüssige Verbindung der Drähte untereinander hergestellt und das Bündelende atraumatisch hergerichtet.

Alternativ können die Drähte parallel geführt und als Fächer an den Stirnseiten miteinander verschmolzen werden. Der Vorteil dieser Ausführung ist der relativ geringe Durchmesser im Anbindungsbereich im Vergleich zur Bündelung der Filamente.

Schließlich ist als weitere Variante die Staffelung der einzelnen Filamente möglich, d. h. die Drähte werden versetzt abgelängt. Jeder Draht wird über seine Stirnfläche mit dem danebenliegenden Draht verbunden. Der längste Draht kann dann die Funktion des Verbinders übernehmen. Die Staffelung kann sowohl mit einer gefächerten als auch mit einer kompakten Führung der Einzeldrähte einhergehen.

Wie vorstehend beschrieben, kommt es bei der spannungsfreien Anordnung der Einzelfilamente im Geflecht darauf an, die Implantatoberfläche möglichst dicht zu gestalten. Da die Flexibilität des Geflechts erhalten bleiben muss, ist eine 100 %ige Oberflächenabdeckung durch die Filamente allerdings allenfalls annähernd möglich. Je nach Anwendung können sich aber auch geringere Oberflächenabdeckungen ergeben bzw. haben sich auch geringere Oberflächenabdeckungen als ausreichend herausgestellt.

Zur Verbesserung der Oberflächenabdeckung kann das Geflecht mit einer Folie ummantelt werden, beispielsweise aus Teflon, Silikon oder einem anderen körperverträglichen Kunststoff. Zur Erhöhung der Flexibilität und Dehnbarkeit kann eine solche Kunststofffolie geschlitzt sein, wobei die Schlitzanordnung gestaffelt ist und die Längsrichtung der Schlitze entlang der Umfangslinie des Implantates verläuft. Eine solche Folie kann beispielsweise durch Eintauchen des Implantates in ein entsprechendes flüssiges Folienmaterial (Dispersion oder Lösung) und anschließende Einfügung der Schlitze, beispielsweise mit einem Laser erzielt werden. Durch Eintauchen kann beispielsweise auch eine ganze oder teilweise Füllung der Maschen erreicht werden.

Alternativ ist es möglich, die einzelnen Filamente des Implantats durch Eintauchen in eine Kunststoffdispersion oder Lösung mit einem solchen Kunststoff zu ummanteln und dadurch den Filamentquerschnitt zu erhöhen. In diesem Fall bleiben offene Maschen, jedoch wird die Maschengröße deutlich vermindert.

Das Implantat gemäß der Erfindung wird aus üblichen Implantatmaterialien mit Rückstelleigenschaften hergestellt, vorzugsweise aus medizinischem Stahl mit Federeigenschaften, Cobalt-Chrom-Legierungen oder einem Material mit Formgedächtniseigenschaften. In letzterem Fall kommt insbesondere Nitinol in Frage. Wichtig ist in jedem Fall, dass das Implantat einerseits in der Lage ist, eine komprimierte Form zwecks Durchführung durch den Einführungskatheter einzunehmen, sich andererseits aber bei Befreiung vom äußeren Zwang des Einführungskatheters automatisch aufweitet und sich am Implantationsort an die Gefäßinnenwandung anlegt. Möglich ist auch die Fertigung des Implantats aus Verbundmaterialien, beispielsweise aus mit Nitinol ummantelten Platindrähten. Auf diese Weise werden die Formgedächtniseigenschaften des Nitinols mit der Röntgensichtbarkeit des Platins kombiniert.

Das Implantat kann auf an und für sich bekannte Weise beschichtet sein. Als Beschichtungsmaterialien kommen insbesondere solche in Frage, wie sie für Stents beschrieben sind, etwa mit antiproliferativen, entzündungshemmenden, antithrombogenen, das Einwachsen fördernden und/oder die Anlagerung hindernden, hämokompatiblen Eigenschaften. Bevorzugt ist eine Beschichtung, die das Einwachsen des Implantats und die Neointimabildung fördert. Es kann sinnvoll sein, das Implantat außen derart zu beschichten und innen mit einem Mittel, das die Anhaftung mindert, etwa Heparin oder einem Derivat, ASS oder dazu geeigneten Oligosacchariden und Chitinderivaten. Hier geeignet sind ferner Schichten aus Nano-Partikeln, etwa ultradünnen Schichten aus polymerem SiO₂, die die Anhaftung mindern.

Erfindungsgemäß können die miteinander verbundenen Filamentenden als Verbindungselemente ausgebildet sein. An diesen Verbindungselementen können die Verdickungen mit definiertem Durchmesser angeordnet sein, wobei diese durch Umschmelzen mit Hilfe eines Lasers erzeugt werden können. Die Verdickungen können eine kugelige, ovale, rechteckige, quadratische o. ä. Form aufweisen und sind dafür vorgesehen, von einem mit einem Einführdraht in Verbindung stehenden Halteelement vor Ablösung des Implantats formschlüssig gehalten zu werden.

An den proximalen und/oder distalen Filamentenden können auch Verbindungselemente angebracht sein, die sich weiter in Richtung proximal/distal erstrecken und an deren Enden sich die Verdickungen befinden. Das Verbindungselement kann bspw. ein Draht sein, der am Verknüpfungspunkt von zwei oder mehr Filamentenden angebracht ist und weiter in axialer Richtung verläuft. Besondere Bedeutung haben die Verdickungen am proximalen Ende, wo die Verdickungen dazu vorgesehen sind, von einem Halteelement formschlüssig gehalten zu werden. Über das Halteelement ist das Implantat an den Einführdraht gekoppelt. Bei Ablösung des Implantats wird der Formschluss zwischen Verdickungen und Halteelement aufgehoben und das Implantat wird freigesetzt. Möglich ist aber auch das zusätzliche Vorsehen eines Halteelements am distalen Ende des Implantats.

Wie schon vorstehend erwähnt, wird die Kombination aus Halteelement und Implantat durch einen endovaskulären Katheter geführt.

Gemäß einer bevorzugten Ausführungsform befinden sich am proximalen Ende des Implantats Verdickungen, die vom Halteelement formschlüssig gehalten werden, wobei ein Abschnitt des Halteelements elektrolytisch korrodierbar ausgebildet ist, so dass nach elektrolytischer Auflösung des Abschnitts das proximale Ende des Implantats freigegeben wird. In diesem Fall wird die Ablösung des Implantats nicht lediglich durch Ausschieben aus dem Katheter bzw. Zurückziehen einer Abdeckung herbeigeführt, stattdessen ist zumindest auch die elektrolytische Korrosion eines Abschnitts des Halteelements notwendig. Der korrodierbare Abschnitt ist dabei so angeordnet, dass er den Austritt der in das Halteelement hineinragenden Verdickungen verhindert. Es kann sich z. B. um einen Stift handeln, der zwischen den Verdickungen angeordnet ist und diese auseinanderhält, so dass der Durchmesser des Implantats am proximalen Ende für einen Austritt aus dem Halteelement zu groß ist. Die Festlegung des Implantats am Halteelement über Formschluss, der über die elektrolytische Ablösbarkeit eines Abschnitts des Halteelements kontrolliert wird, ist im Hinblick auf die sichere Steuerbarkeit der Platzierung und ggf. auch die Repositionierung oder das Zurückziehen des Implantats besonders vorteilhaft. Selbstverständlich ist aber auch eine reine elektrolytische Ablösbarkeit des Implantats vom Halteelement denkbar, wie sie im Stand der Technik für Stents und Coils bekannt ist.

Eine weitere Möglichkeit besteht darin, als korrodierbar ausgebildeten Abschnitt des Halteelements eine Scheibe mit einer Öffnung zu verwenden, wobei sich die am proximalen Ende des Implantats befindlichen Verdickungen durch die Öffnung erstrecken und wobei der Durchmesser der Öffnung so auf die Verdickungen abgestimmt ist, dass ein Durchtreten der Verdickungen durch die Öffnung bei intakter Scheibe ausgeschlossen ist. Erst wenn sich die Scheibe durch Anlegen einer Spannung zumindest teilweise auflöst, können die Verdickungen des Implantats aus dem Halteelement austreten.

Für den elektrolytisch korrodierbaren Abschnitt können verschiedene Materialien verwendet werden, die eine rasche Ablösbarkeit gewährleisten und darüber hinaus medizinisch verträglich sind. Beispiele sind Edelstahl, Magnesium, Magnesiumlegierungen oder Cobalt-Chrom-Legierungen.

Das Halteelement kann aus zwei zueinander beabstandeten Fixierelementen bestehen, die das Implantat zwischen sich gespannt aufnehmen. In diesem Fall weisen beide Fixierelemente die entsprechenden Aufnahmen für die Verbindungselemente des Implantats und das Implantat entsprechende Verbindungselemente sowohl an seinem proximalen wie auch an seinem distalen Ende auf.

Ein entsprechend ausgebildetes Halteelement mit zwei Fixierelementen kann die beiden Fixierelemente in einem definierten Abstand an ein und demselben Einführdraht aufweisen, wodurch das Implantat bei vorgegebener Länge auch eine definierte Streckung und Spannung erfährt. Auf diese Art und Weise ist sichergestellt, dass keine Überdehnung stattfindet und die Rückstellkräfte nach der Freisetzung im Gefäß voll wirksam werden können. Alternativ ist aber auch eine Fixierung der Fixierelemente an zwei separaten Einführdrähten möglich, die eine Einstellung oder Streckung des Implantats durch den behandelnden Arzt oder über eine entsprechende Feststellvorrichtung ermöglicht. Der zweite Einführdraht kann auch als Führungsrohr ausgebildet sein.

Gemäß einer weiteren vorteilhaften Ausführungsform verfügt der Einführdraht über eine Führungsdrahtspitze, die sich vom distalen Ende des Einführdrahtes weiter in Richtung distal in den Innenraum des Implantats erstreckt, insbesondere bis zum distalen Ende des Implantats oder darüber hinaus. Auf diese Weise wird bewirkt, dass auch nach Freisetzung des Implantats zunächst noch ein Objekt durch das Innere des Implantats verläuft, solange der Einführdraht nicht zurückgezogen wird. Dies ermöglicht die erneute Sondierung des Gefäßes bzw. Implantates, beispielsweise indem ein Katheter über den Einführdraht und daran anschließend die Führungsdrahtspitze geführt wird. Der Katheter wird auf diese Weise durch das freigesetzte und expandierte Implantat bewegt. Die Führungsdrahtspitze wird erst durch das endgültige Zurückziehen des Einführdrahtes entfernt.

Die Führungsdrahtspitze kann ein rotationssymmetrisches Design haben. Der Querschnitt kann rund, oval, rechteckig oder auf grundsätzlich beliebige andere Art geformt sein. Sinnvoll ist es darüber hinaus, die Führungsdrahtspitze visualisierbar zu machen, z. B. indem die Führungsdrahtspitze selbst zumindest teilweise aus einem röntgensichtbaren Material gefertigt wird oder indem die Führungsdrahtspitze an ihrem distalen Ende einen röntgendichten Marker aufweist. Hergestellt werden kann die Führungsdrahtspitze aus Edelstahl, Nitinol oder anderen Metallen.

Die Führungsdrahtspitze und der eigentliche Einführdraht können einstückig gefertigt sein, d. h. es handelt es sich letztlich um einen durchgehenden Draht. Möglich ist es aber auch, Führungsdrahtspitze und Einführdraht getrennt zu fertigen und erst nachträglich miteinander zu verbinden. Zumeist wird die Führungsdrahtspitze einen kleineren Durchmesser aufweisen als der Führungsdraht, d. h. der Querschnitt verringert sich vom Einführdraht zur Führungsdrahtspitze hin. Weiter vorteilhaft ist es darüber hinaus, wenn die Führungsdrahtspitze leicht konisch gestaltet ist, d. h. ihr Durchmesser in Richtung distal abnimmt, so dass sich die Flexibilität in Richtung distal erhöht.

Der Erfindung wird durch die nachfolgenden Darstellungen näher erläutert. Es zeigt:
- Figur 1: Ein typisches Beispiel eines Geflechts, wie es erfindungsgemäß zum Einsatz kommt;
- Figur 2: Filamente mit einfacher und zweifacher Fachung;
- Figur 3: ein einflechtiges und ein zweiflechtiges Geflecht;
- Figur 4: die Art und Weise der Zusammenführung der Filamentenden eines erfindungsgemäßen Geflechts;
- Figur 5a: eine alternative Ausführungsform zur Verbindung der Filamentenden;
- Figur 5b: die atraumatische Ausbildung der Filamentenden;
- Figur 6: eine weitere alternative Ausführungsform zur Verbindung der Filamentenden;
- Figur 7: die Fixierung der Kreuzungspunkte;
- Figur 8: eine Ausführungsform mit zusätzlichen Verbindungselementen;
- Figur 9: eine alternative Ausführungsform zur Fixierung der Kreuzungspunkte;
- Figur 10: eine Ausführungsform mit axial versetzten Hülsen;
- Figur 11: eine nicht erfindungsgemäße Form der Anbindung eines Implantats am Halteelement sowie die Ablösung;
- Figur 12: eine erfindungsgemäße Form der Ablösung des Implantats vom Halteelement; und
- Figur 13: eine weitere erfindungsgemäße Form der Ablösung des Implantats vom Halteelement und
- Figur 14: eine nicht erfindungsgemäße Ausgestaltung mit durch das Implantat verlaufender Führungsdrahtspitze.

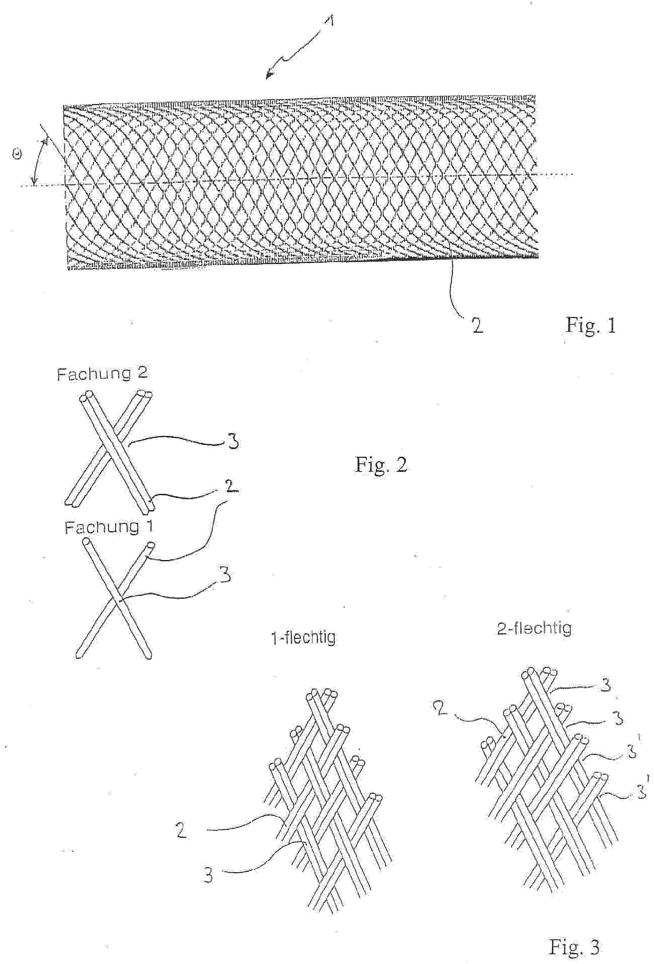

Figur 1 zeigt die Geflechtstruktur eines erfindungsgemäßen Implantats 1, das aus miteinander verflochtenen Filamenten 2 besteht. Die einzelnen Filamente kreuzen sich im gezeigten Fall in einem Winkel von etwa 120°, wobei die offenen Seiten des Winkels zu den offenen Enden des Geflechts weisen. Das Geflecht ist in leicht gespanntem/gelängtem Zustand dargestellt, d. h. mit einem reduzierten Durchmesser.

Der Winkel Theta bezeichnet den Geflechtswinkel zur Längsachse, der im entspannten Zustand beim Nenndurchmesser bis zu 80° betragen kann. Bei der Längung des Geflechts im Katheter kann der Winkel Theta bis auf ca. 7° abnehmen.

Es versteht sich, dass der Nenndurchmesser des Geflechts auf das Lumen des Zielgefäßes an der zu behandelnden Stelle abgestimmt ist.

Das Geflecht wird auf einer konventionellen Flechtmaschine hergestellt und liegt als Endlosgeflecht vor. Geflochten wird auf einem Dorn, dessen äußere Abmessungen dem Innendurchmesser der späteren Produkte entsprechen.

Die Flechtmaschine und deren Besetzung bestimmt die Struktur des Geflechts, z. B. die Anzahl der Fäden, den Fadenverlauf und die Anzahl der Kreuzungspunkte am Umfang und per Schlaglänge. Die Anzahl der Fäden ist abhängig von der Zahl der Klöppel, wobei die Klöppel je zur Hälfte in beide Richtungen um den Flechtkern laufen.

Die Filamente bestehen in der Regel aus Metall, beispielsweise aus Stahldraht, röntgendichten Platinmetallen oder -legierungen oder Nitinol. Es können auch Kunststofffäden, die über die nötige Flexibilität verfügen, verwendet werden. Die Filamentstärke beträgt idealerweise 0,01 bis 0,2 mm, insbesondere 0,02 bis 0,1 mm. Um eine hohe Abdeckung der Wandfläche zu erzielen, kann anstelle von Drahtmaterial auch ein Flachbandmaterial verwandt werden, beispielsweise mit einer Breite von 0,05 bis 0,5 mm, vorzugsweise bis 0,1 mm, bei den oben angegebenen Stärken.

Das erfindungsgemäße Geflecht kann aus Einzelfilamenten hergestellt werden (Fachung 1) oder auch aus zwei (Fachung 2) oder mehr Einzelfilamenten.

Figur 2 zeigt Kreuzungspunkte 3, an denen sich jeweils zwei parallel geführte Filamente kreuzen (Fachung 2) oder nur Einzelfilamente 2 kreuzen (Fachung 1). Werden zwei oder mehr Filamente zusammengefasst, werden diese über ein und dieselbe Spule zugeführt.

Figur 3 zeigt Muster für einflechtige und zweiflechtige Strukturen aus Filamenten 2 der Fachung 2. In der einflechtigen Struktur liegen die Filamentpaare alternierend übereinander und untereinander. Bei der zweiflechtigen Struktur werden die Filamentpaare, wie dargestellt, jeweils über zwei gegenläufige Filamentpaare geführt, bevor sie unter zwei gegenläufigen Filamentpaaren hindurchgeführt werden.

Eine Fachung von zwei oder eine noch höhere Fachung bringt eine höhere Oberflächendichte des Geflechts mit sich, bei gleichzeitiger Verringerung der Längenausdehnung während der Komprimierung des Geflechts. Diese höhere Oberflächendichte geht allerdings auf Kosten der Flexibilität, auch durch Erhöhung der Reibung und Spannung. Dem kann durch eine Erhöhung der Flechtigkeit entgegengewirkt werden, d. h. eine zwei oder höherflechtige Struktur bringt eine Erhöhung der Flexibilität mit sich. Erfindungsgemäß ist eine Flechtigkeit von 2 und eine Fachung von 2 bevorzugt.

Nach der Auftrennung in für das Produkt spezifische Einheiten muss das Geflecht an den Enden geschlossen werden. Dies ist notwendig, um die Formstabilität des Flechtkörpers zu gewährleisten und um Gefäßverletzungen zu vermeiden. Hierfür ist auch die Ordnung der Struktur an den Enden des Geflechts notwendig.

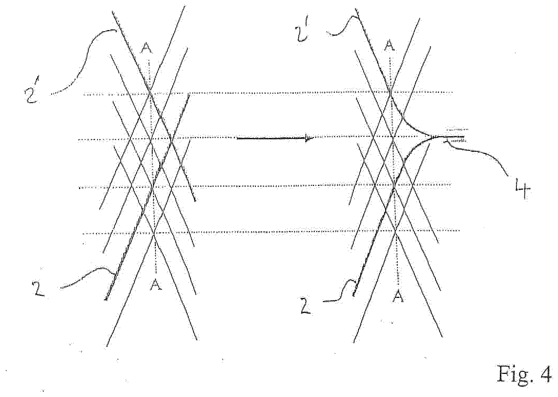

Figur 4 zeigt die Zusammenführung zweier Filamente 2, 2' am Ende des Geflechts zu einem Filamentpaar 4, wobei 2 und 2' gegenläufige Filamente sind. Die Filamente werden dazu in axialer Richtung umgebogen und distal miteinander verschweißt. Dabei werden jeweils die in den randständigen Kreuzungspunkten übereinanderliegenden Filamente miteinander verbunden. Solche Kreuzungspunkte befinden sich beispielsweise auf der Linie A-A.

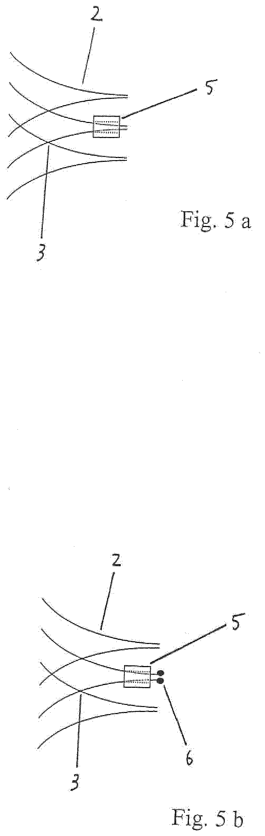

In Figur 5a ist gezeigt, wie die Enden der Filamente 2 durch eine Hülse 5 zusammengehalten werden. Die Hülse 5 kann mit den Filamenten verschweißt oder vercrimpt sein. Darüber hinaus kann die Hülse 5 gleichzeitig der Visualisierung des Implantationsvorgangs dienen, wenn sie aus einem röntgendichten/röntgensichtbaren Material gefertigt ist.

Wie in Figur 5b dargestellt, können die Filamentenden atraumatische Verdickungen 6 aufweisen. Diese können aus dem Filament 2 selbst gebildet oder zusätzlich angebracht werden. Wenn die Verdickungen 6 einen ausreichenden Durchmesser aufweisen, wird die Hülse 5 allein dadurch davon abgehalten, von den Filamentenden abzurutschen. Ebenso möglich ist aber selbstverständlich die Festlegung der Hülse 5 durch Vercrimpen, Verschweißen, Löten, Kleben o. ä.. Die Figuren 5a,b zeigen das distale Ende eines Implantats 1, möglich ist aber auch eine ähnliche Filamentfestlegung am proximalen Ende.

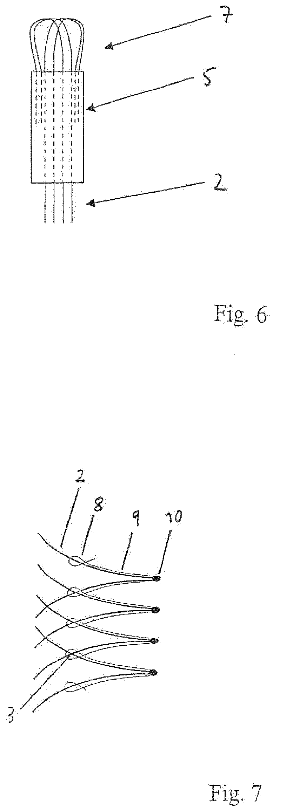

Figur 6 zeigt ebenfalls die Festlegung von Enden der Filamente 2 durch eine Hülse 5, insbesondere am distalen Ende. Hierbei werden aber im Gegensatz zum zuvor dargestellten Beispiel die Enden der Filamente so zurück in die Hülse 5 geführt, dass sich eine Schlaufe 7 bildet. Auch auf diese Weise kann ein atraumatisches Ende gebildet werden.

In Figur 7 wird die gegenseitige Fixierung der sich kreuzenden Filamente 2 am proximalen Kreuzungspunkt 3 dargestellt. Dies geschieht, indem um den Kreuzungspunkt 3 eine Schlaufe 8 gelegt wird. Diese Schlaufe 8 wird vom Draht 9 gebildet, der wiederum am proximalen Ende 10 des Implantats 1 festgelegt ist. Auf diese Weise wird bei der Aufweitung des Implantats 1 eine Verlagerung der am weitesten proximal gelegenen Kreuzungspunkte 3 verhindert, was eine vollständige Aufweitung gewährleistet, ohne dass einzelne Filamente 2 nach innen in das Gefäßlumen hinein stehen.

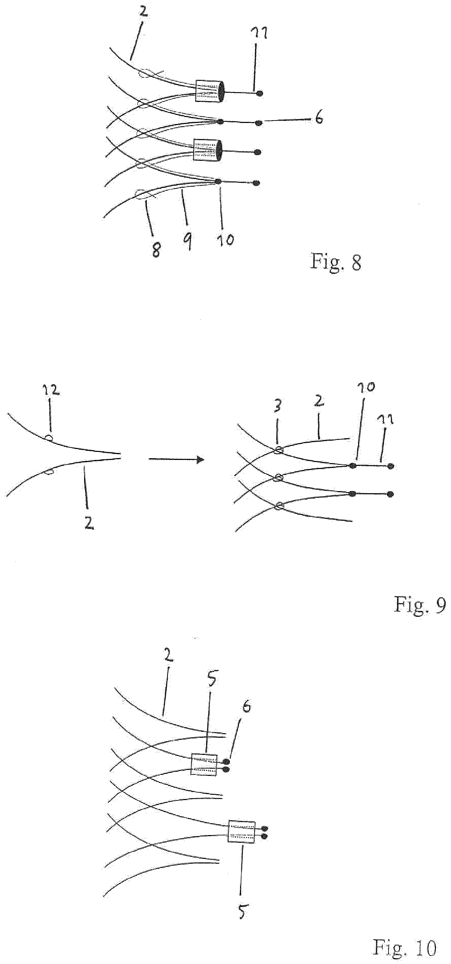

In Figur 8 ist die Ausführungsform aus Figur 7 um zusätzliche Hülsen 5 ergänzt, wobei in diesem Fall die Hülsen 5 nur auf einigen Filamentenden angebracht sind. Die Hülsen 5 dienen hier als Markierungshülsen und sind aus einem röntgendichten Material gefertigt, um die Einbringung des Implantats 1 unter Röntgenkontrolle zu erlauben.

Zusätzlich zeigt die Figur 8 am proximalen Ende des Implantats 1 angebrachte Verbindungselemente 11, die seinerseits am proximalen Ende Verdickungen 6 aufweisen. Diese Verdickungen 6 sind für den Eingriff in ein Halteelement 15 geeignet, das der Kontrolle der Freisetzung des Implantats 1 dient.

In Figur 9 ist eine alternative Möglichkeit dargestellt, die Filamente 2 im Bereich der proximalen Kreuzungspunkte 3 miteinander zu verbinden. Die Filamente verfügen im Bereich der proximalen Kreuzungspunkte 3 über Ösen 12. Das Filament 2, das sich mit dem eine Öse 12 aufweisenden Filament 2 in diesem Bereich kreuzt, verläuft durch die Öse 12, wobei auch dieses Filament 2 zur Vermeidung der Längsverschieblichkeit an dieser Stelle über eine Öse 12 verfügt. Die Mittel zur Fixierung der Filamente 2 sind somit in diesem Fall Teil der Filamente 2 selbst.

In Figur 10 ist das proximale oder distale Ende eines Implantats 1 dargestellt, wobei Hülsen 5 als röntgendichte Marker auf die Filamentbündel aufgesetzt sind. Die Hülsen 5 sind axial in gewissem Maße versetzt. Auf diese Weise wird erreicht, dass die radiale Ausdehnung des Implantats 1 in komprimiertem Zustand klein gehalten wird, d. h. die Profilhöhe ist geringer als bei Anbringung sämtlicher Hülsen 5 an der gleichen axialen Position.

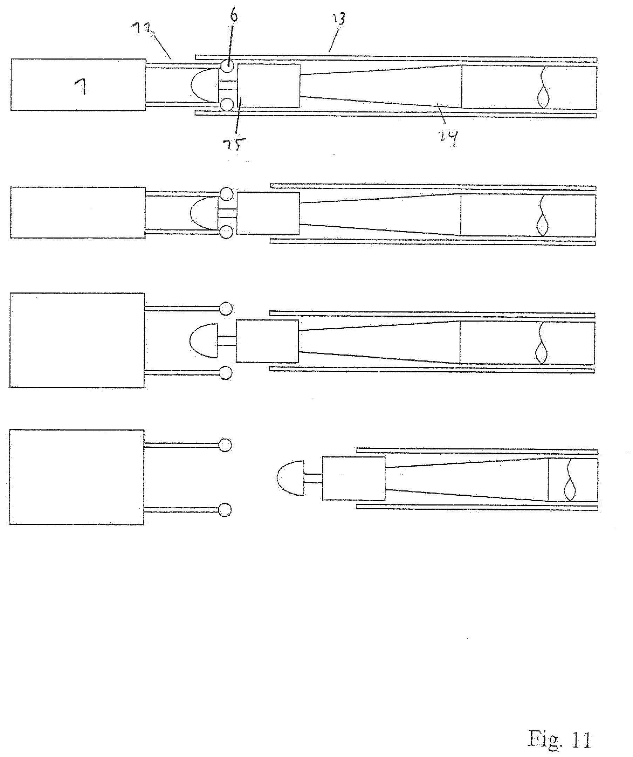

Die Figur 11 zeigt die nicht erfindungsgemäße Festlegung und Ablösung des Implantats 1, das über ein Halteelement 15 mit einem Einführdraht 14 verbunden ist. Halteelement 15 und Einführdraht 14 sind von einer schlauchförmigen Abdeckung 13 umhüllt. Das Halteelement 15 verfügt über Ausnehmungen, in die die Verdickungen 6 am proximalen Ende des Implantats 1 eingreifen. Solange die Abdeckung 13 das Halteelement umgibt, können die Verdickungen 6 nicht aus dem Halteelement 15 heraustreten. Sobald jedoch die Abdeckung 13 zurückgezogen wird, kann das Implantat 1 am proximalen Ende expandieren und die Verdickungen lösen sich aus den Ausnehmungen im Halteelement 15. Anschließend wird auch der Einführdraht 14, an dessen distalem Ende sich das Halteelement 15 befindet, zurückgezogen.

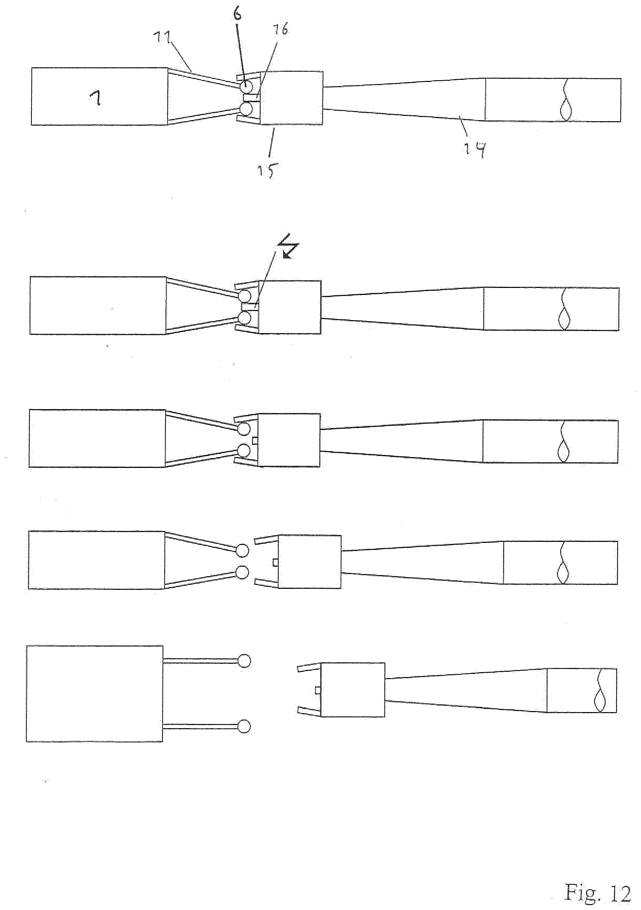

In Figur 12 ist eine erfindungsgemäße Ausführungsform zur Ablösung des Implantats 1 vom Halteelement 15 gezeigt, bei der ebenfalls an den Verbindungselementen 11 angebrachte Verdickungen 6 in entsprechende Ausnehmungen des Halteelements 15 eingreifen, die Freisetzung erfolgt aber nicht über das Zurückziehen einer Abdeckung, sondern dadurch, dass der elektrolytisch korrodierbar ausgebildete Abschnitt 16 durch Anlegen einer elektrischen Spannung, symbolisiert durch einen Blitz, aufgelöst wird. Dieser Abschnitt 16 behindert vor der Auflösung den Austritt der Verdickungen 6 aus dem Halteelement. Nach Auflösung hingegen steht ausreichend Platz zur Verfügung, so dass sich das Implantat 1 ablösen und aufweiten kann. Die Kombination von formschlüssiger Festlegung des Implantats 1 am Halteelement 15 mit elektrolytischer Ablösbarkeit erlaubt es, auf eine zusätzliche Abdeckung oder Umhüllung des Halteelements 15 zu verzichten.

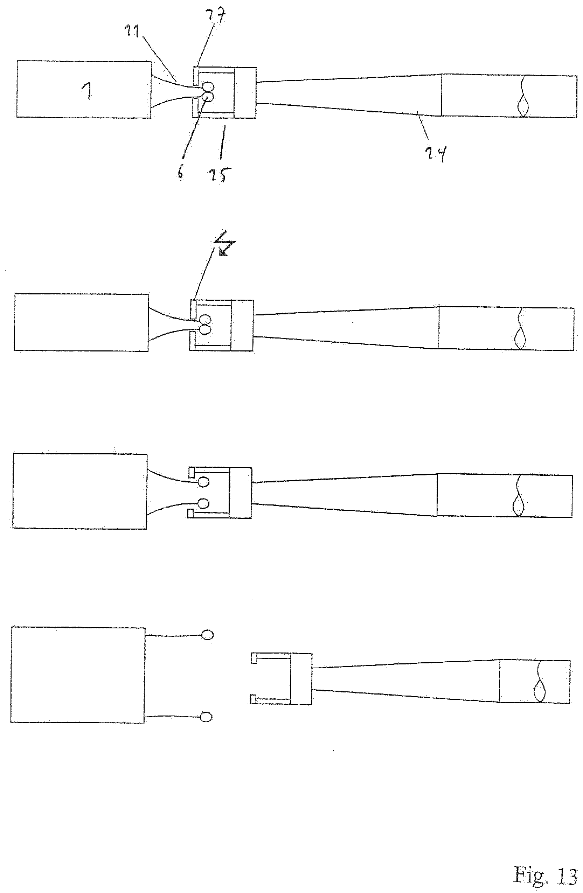

Eine weitere Möglichkeit der elektrolytischen Ablösbarkeit ist in Figur 13 dargestellt. Hier werden die Verdickungen 6 formschlüssig im Halteelement 15 gehalten, wobei eine Scheibe 17 mit einer Öffnung in der Mitte das Austreten der Verdickungen 6 verhindert. Die Öffnung hat dabei einen Durchmesser, der zwar die Durchführung der Verbindungselemente 11, nicht aber den Durchtritt der Verdickungen 6 am proximalen Ende der Verbindungselemente 11 erlaubt. Sobald jedoch die Scheibe 17 elektrolytisch aufgelöst wird, kann sich das Implantat 1 lösen und aufweiten. Anschließend wird der Einführdraht 14 mit dem Halteelement 15 zurückgezogen.

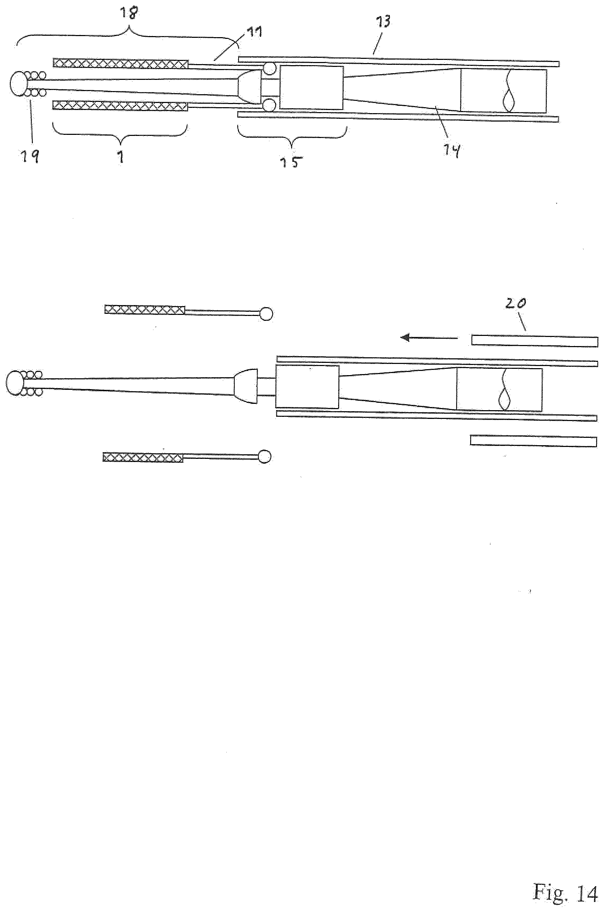

In Figur 14 ist ein nicht erfindungsgemäßes Beispiel dargestellt, bei dem sich ein Führungsdrahtabschnitt, nämlich die sich distal an den Einführdraht 14 anschließende Führungsdrahtspitze 18, durch das Innere des Implantates 1 erstreckt. Die Führungsdrahtspitze 18 verläuft durch das gesamte Implantat 1 und schließt am distalen Ende mit einem Marker 19 aus röntgendichtem Material ab, welcher hier als Markercoil ausgebildet ist. Die Führungsdrahtspitze 18 ist dünner als der Einführdraht 14 selbst und läuft in Richtung distal konisch zu, wodurch zum einen sichergestellt wird, dass auch der Innenraum des Implantats 1 in komprimierter Form für die Führungsdrahtspitze 18 ausreichend groß ist, und zum anderen, dass sich die Flexibilität in Richtung distal erhöht.

Durch Zurückziehen der Abdeckung 13 wird, wie im Zusammenhang mit Figur 11 beschrieben, das Implantat 1 freigesetzt, so dass eine Expansion des Implantats 1 erfolgt. Gleichwohl verläuft die Führungsdrahtspitze 18 weiterhin durch das Innere des Implantats 1, solange der Einführdraht 14 nicht zurückgezogen wird. Aufgrund der Expansion und der damit verbundenen Verkürzung des Implantats 1 ragt die Führungsdrahtspitze 18 nach der Freisetzung noch etwas weiter über das distale Ende des Implantats 1 heraus als zuvor. Nunmehr kann, sofern aus Sicht des Arztes erforderlich, ein Katheter 20 über den Einführdraht 14 und die Führungsdrahtspitze 18 durch das Implantat 1 geschoben werden, wie durch den Pfeil angedeutet wird.

## Patentansprüche

1. Kombination aus
- einem Implantat (1) für Blutgefäße, insbesondere zur Beeinflussung des Blutflusses im Bereich arteriovenöser Fehlbildungen, mit einer Wandung aus einzelnen Filamenten (2), die zu einem im Wesentlichen röhrenförmigen Geflecht zusammengefasst sind, das sich in axialer Richtung von proximal nach distal erstreckt, wobei die einzelnen Filamente (2) sich gegenseitig kreuzen und miteinander Kreuzungspunkte (3) bilden und das Implantat (1) in der Weise verformbar ist, dass es in einem Einführungskatheter eine Form mit vermindertem Durchmesser annimmt und am Ort der Implantation unter Anpassung an den Blutgefäßdurchmesser expandiert, und
- einem Einführdraht (14), wobei das Implantat (1) über ein Halteelement (15) an den Einführdraht (14) gekoppelt ist, und am proximalen und/oder distalen Ende des Geflechts die Filamentenden jeweils mindestens paarweise zusammengeführt und miteinander dauerhaft verbunden sind, wobei die miteinander verbundenen Filamentenden atraumatisch geformt sind, charakterisiert dadurch, dass sich am proximalen Ende des Implantats (1)
Verdickungen (6) befinden, die vom Halteelement (15) formschlüssig gehalten werden, wobei ein Abschnitt (16) des Halteelements (15) elektrolytisch korrodierbar ausgebildet und durch Anlegen einer elektrischen Spannung auflösbar ist, wobei der Abschnitt (16) vor der Auflösung den Austritt der Verdickungen (6) aus dem Halteelement (15) behindert und nach elektrolytischer Auflösung des Abschnitts (16) für den Austritt der Verdickungen (6) ausreichend Platz zur Verfügung steht, so dass sich das Implantat (1) ablösen und aufweiten kann.

2. Kombination nach Anspruch 1, **dadurch gekennzeichnet, dass** der elektrolytisch korrodierbar ausgebildete Abschnitt (16) ein Stift ist, der zwischen den Verdickungen (6) angeordnet ist und diese auseinanderhält.

3. Kombination nach Anspruch 1, **dadurch gekennzeichnet, dass** der korrodierbar ausgebildete Abschnitt (16) des Halteelements (15) eine Scheibe (17) mit einer Öffnung ist, wobei sich die am proximalen Ende des Implantats (1) befindlichen Verdickungen (6) durch die Öffnung erstrecken und wobei der Durchmesser der Öffnung so auf die Verdickungen (6) abgestimmt ist, dass ein Durchtreten der Verdickungen (6) durch die Öffnung bei intakter Scheibe (17) ausgeschlossen ist.

4. Kombination nach einem der Ansprüche 1 bis 3, **dadurch gekennzeichnet, dass** der korrodierbar ausgebildete Abschnitt (16) aus Edelstahl, Magnesium, Magnesiumlegierungen oder Cobalt-Chrom-Legierungen ist.

5. Kombination nach einem der Ansprüche 1 bis 4, **dadurch gekennzeichnet, dass** die sich kreuzenden Filamente (2) an den distal der Filamentenden liegenden Kreuzungspunkten (3) am proximalen Ende (10) des Implantats (1) jeweils direkt oder indirekt miteinander verbunden sind.

6. Kombination nach Anspruch 5, **dadurch gekennzeichnet, dass** die sich kreuzenden Filamente (2) an den am weitesten proximal liegenden Kreuzungspunkten (3) distal der Filamentenden jeweils direkt oder indirekt miteinander verbunden sind.

7. Kombination nach Anspruch 5 oder 6, **dadurch gekennzeichnet, dass** die Filamente (2) an den am proximalen Ende (10) des Implantats (1) liegenden Kreuzungspunkten (3) durch Schlaufen (8) geführt sind, welche von am Implantat (1) festgelegten Drähten (9) gebildet werden.

8. Kombination nach Anspruch 7, **dadurch gekennzeichnet, dass** die die Schlaufen (8) bildenden Drähte (9) am proximalen Ende (10) des Implantats (1) festgelegt sind.

9. Kombination nach Anspruch 5 oder 6, **dadurch gekennzeichnet, dass** zumindest einige Filamente (2) an den am proximalen Ende (10) des Implantats (1) liegenden Kreuzungspunkten (3) Ösen (12) bilden, durch die die sich mit dem jeweiligen Filament (2) an diesem Kreuzungspunkt (3) kreuzenden weiteren Filamente (2) verlaufen.

10. Kombination nach Anspruch 5 oder 6, **dadurch gekennzeichnet, dass** die sich im Bereich der am proximalen Ende (10) des Implantats (1) liegenden Kreuzungspunkte (3) kreuzenden Filamente (2) an dieser Stelle miteinander verknotet, verklebt, verlötet oder verschweißt sind.

11. Kombination nach einem der Ansprüche 1 bis 10, **dadurch gekennzeichnet, dass** sich an den am proximalen und/oder distalen Ende des Implantats (1) zusammengeführten Enden der Filamente (2) röntgendichte Markierungen befinden.

12. Kombination nach Anspruch 11, **dadurch gekennzeichnet, dass** die röntgendichten Markierungen Hülsen (5) sind, die die zusammengeführten Filamente (2) umschließen.

13. Kombination nach Anspruch 12, **dadurch gekennzeichnet, dass** die Hülsen (5), die benachbarte zusammengeführte Filamente (2) umschließen, in axialer Richtung gegeneinander versetzt sind.

14. Kombination nach einem der Ansprüche 1 bis 13, **dadurch gekennzeichnet, dass** der Einführdraht (14) über eine Führungsdrahtspitze (18) verfügt, die sich vom distalen Ende des Einführdrahtes (14) weiter in Richtung distal in den Innenraum des Implantats (1) erstreckt, insbesondere bis zum distalen Ende des Implantats (1) oder darüber hinaus.

## Claims

1. Combination of
- an implant (1) for blood vessels, in particular for influencing the blood flow in the region of arteriovenous defects, comprising a wall consisting of individual filaments (2) which are combined to form a substantially tubular lattice which extends in the axial direction from a proximal side to a distal side, wherein the individual filaments (2) cross one another and form crossing points (3) with one another, and the implant (1) is deformable such that it assumes a shape with a reduced diameter in an insertion catheter and expands over the implantation site, adapting to the diameter of the blood vessel, and
- an insertion wire (14), wherein the implant (1) is coupled to the insertion wire (14) via a retaining element (15) and, at the proximal and/or distal end of the lattice, the filament ends are brought together in each case at least in pairs and permanently connected to one another, wherein the filament ends connected to one another are formed atraumatically, **characterised in that** at the proximal end of the implant (1) there are bulges (6) which are held in a form-fitting manner by the retaining element (15), wherein a portion (16) of the retaining element (15) is designed to be electrolytically corrodible and dissolvable by applying an electrical voltage, wherein the portion (16) inhibits the bulges (6) from emerging from the retaining element (15) before the dissolution and, after the electrolytical dissolution of the portion (16), sufficient space is available for the emergence of the bulges (6) so that the implant (1) can detach and expand.

2. Combination according to claim 1, **characterised in that** the electrolytically corrodible portion (16) is a pin which is arranged between the bulges (6) and holds them apart from one another.

3. Combination according to claim 1, **characterised in that** the portion (16) of the retaining element (15) which is designed to be corrodible is a disc (17) having an opening, wherein the bulges (6) located at the proximal end of the implant (1) extend through the opening and wherein the diameter of the opening is adapted to the bulges (6) in such a way that passage of the bulges (6) through the opening is excluded when the disc (17) is intact.

4. Combination according to any one of claims 1 to 3, **characterised in that** the portion (16) designed to be corrodible is made of stainless steel, magnesium, magnesium alloys or cobalt-chromium alloys.

5. Combination according to any one of claims 1 to 4, **characterised in that** the filaments (2) crossing each other at the crossing points (3) located distally of the filament ends at the proximal end (10) of the implant (1) are each connected directly or indirectly to one another.

6. Combination according to claim 5, **characterised in that** the filaments (2) crossing each other at the crossing points (3) furthest away in the proximal direction distal of the filament ends are each connected directly or indirectly to one another.

7. Combination according to any one of claims 5 to 6, **characterised in that** the filaments (2) at the crossing points (3) located at the proximal end (10) of the implant (1) are guided through loops (8) which are formed by wires (9) fixed to the implant (1).

8. Combination according to claim 7, **characterised in that** the wires (9) forming the loops (8) are fixed to the proximal end (10) of the implant (1).

9. Combination according to claim 5 or 6, **characterised in that** at least some filaments (2) at the crossing points (3) located at the proximal end (10) of the implant (1) form eyelets (12) through which the other filaments (2) crossing with the respective filament (2) at this crossing point (3) extend.

10. Combination according to claim 5 or 6, **characterised in that** the filaments (2) crossing each other in the region of the crossing points (3) located at the proximal end (10) of the implant (1) are knotted, glued, brazed or welded together at this point.

11. Combination according to any one of claims 1 to 10, **characterised in that** radiopaque markings are located at the ends of the filaments (2) brought together at the proximal and/or distal end of the implant (1).

12. Combination according to claim 11, **characterised in that** the radiopaque markings are sleeves (5) which enclose the filaments (2) brought together.

13. Combination according to claim 12, **characterised in that** the sleeves (5) enclosing adjacent filaments (2) are offset relative to one another in the axial direction.

14. Combination according to any one of claims 1 to 13, **characterised in that** the insertion wire (14) has a guide wire tip (18) which extends from the distal end of the insertion wire (14) further in the distal direction into the interior of the implant (1), in particular to the distal end of the implant (1) or beyond.

## Revendications

1. Combinaison de
- un implant (1) pour vaisseaux sanguins, en particulier destiné à influencer la circulation sanguine dans la zone de malformations artério-veineuses, comportant une paroi constituée de filaments (2) individuels, qui sont rassemblés en un treillis sensiblement tubulaire, qui s'étend dans la direction axiale d'un côté proximal à un côté distal, les filaments (2) individuels se croisant mutuellement et formant les uns avec les autres des points de croisement (3) et l'implant (1) pouvant être déformé de manière à atteindre une forme ayant un diamètre réduit dans un cathéter d'introduction et à s'étendre sur le site de l'implantation en s'adaptant au diamètre du vaisseau sanguin, et
- un fil d'introduction (14), l'implant (1) étant accouplé au fil d'introduction (14) par le biais d'un élément de retenue (15) et, à l'extrémité proximale et/ou distale du treillis, les extrémités de filament étant rassemblées respectivement au moins par paires et reliées les unes aux autres de manière permanente, les extrémités de filament reliées les unes aux autres étant formées de manière atraumatique, **caractérisé en ce que** des renflements (6), maintenus par complémentarité de forme par l'élément de retenue (15), se trouvent à l'extrémité proximale de l'implant (1), une partie (16) de l'élément de retenue (15) étant réalisée de manière corrodable par voie électrolytique et pouvant être dissoute par l'application d'une tension électrique, la partie (16) empêchant les renflements (6) de sortir de l'élément de retenue (15) avant la dissolution et, après la dissolution électrolytique de la partie (16), suffisamment de place étant disponible pour la sortie des renflements (6) de telle sorte que l'implant (1) puisse se détacher et s'élargir.

2. Combinaison selon la revendication 1, **caractérisée en ce que** la partie (16) réalisée de manière corrodable par voie électrolytique est un ergot qui est disposé entre les renflements (6) et maintient ceux-ci à l'écart l'un de l'autre.

3. Combinaison selon la revendication 1, **caractérisée en ce que** la partie (16) de l'élément de retenue (15) réalisée de manière corrodable est un disque (17) doté d'une ouverture, les renflements (6) situés à l'extrémité proximale de l'implant (1) s'étendant à travers l'ouverture et le diamètre de l'ouverture étant adapté aux renflements (6) de telle sorte qu'un passage des renflements (6) à travers l'ouverture est exclu lorsque le disque (17) est intact.

4. Combinaison selon l'une des revendications 1 à 3, **caractérisée en ce que** la partie (16) réalisée de manière corrodable est en acier fin, en magnésium, en alliages de magnésium ou en alliages cobalt-chrome.

5. Combinaison selon l'une des revendications 1 à 4, **caractérisée en ce que** les filaments (2) se croisant aux points de croisement (3) situés côté distal des extrémités de filament à l'extrémité proximale (10) de l'implant (1) sont reliés respectivement directement ou indirectement les uns aux autres.

6. Combinaison selon la revendication 5, **caractérisée en ce que** les filaments (2) se croisant aux points de croisement (3) situés le plus loin dans la direction proximale du côté distal des extrémités de filament sont reliés respectivement directement ou indirectement les uns aux autres.

7. Combinaison selon l'une des revendications 5 à 6, **caractérisée en ce que** les filaments (2) aux points de croisement (3) situés à l'extrémité proximale (10) de l'implant (1) sont guidés à travers des boucles (8) qui sont formées par des fils (9) fixés sur l'implant (1).

8. Combinaison selon la revendication 7, **caractérisée en ce que** les fils (9) formant les boucles (8) sont fixés à l'extrémité proximale (10) de l'implant (1).

9. Combinaison selon la revendication 5 ou 6, **caractérisée en ce qu'**au moins quelques filaments (2) aux points de croisement (3) situés à l'extrémité proximale (10) de l'implant (1) forment des oeillets (12) à travers lesquels s'étendent les autres filaments (2) se croisant avec le filament (2) respectif en ce point de croisement (3).

10. Combinaison selon la revendication 5 ou 6, **caractérisée en ce que** les filaments (2) se croisant dans la zone des points de croisement (3) situés à l'extrémité proximale (10) de l'implant (1) sont noués, collés, brasés ou soudés ensemble en ce point.

11. Combinaison selon l'une des revendications 1 à 10, **caractérisée en ce que** des marquages radio-opaques se trouvent aux extrémités des filaments (2) rassemblées à l'extrémité proximale et/ou distale de l'implant (1).

12. Combinaison selon la revendication 11, **caractérisée en ce que** les marquages radio-opaques sont des manchons (5) qui entourent les filaments (2) rassemblés.

13. Combinaison selon la revendication 12, **caractérisée en ce que** les manchons (5) qui entourent des filaments (2) rassemblés adjacents sont décalés les uns par rapport aux autres dans la direction axiale.

14. Combinaison selon l'une des revendications 1 à 13, **caractérisée en ce que** le fil d'introduction (14) dispose d'une pointe de fil de guidage (18) qui s'étend depuis l'extrémité distale du fil d'introduction (14), dans la direction distale jusqu'à l'intérieur de l'implant (1), en particulier jusqu'à l'extrémité distale de l'implant (1) ou au-delà.
